# EUROPEAN PATENT APPLICATION

(11) **EP 4 317 453 A1**
(43) Date of publication of application: **07.02.2024**
(21) Application number: 22780281.6
(22) Date of filing: 22.03.2022
(51) Int. Cl.: C12Q 1/68, G01N 33/53

(54) **ANTIDRUG ANTIBODY MEASUREMENT METHOD**

(30) Priority: 31.03.2021 JP 2021061507
(71) Applicant: Sekisui Medical Co., Ltd., Tokyo 103-0027 (JP)
(72) Inventor: SEKINO, Tetsuo, Tokyo 103-0027 (JP); OOMORI, Akane, Tokyo 103-0027 (JP)
(74) Representative: Hoffmann Eitle
(86) International application number: PCT/JP2022/012965
(87) International publication number: WO 2022/210066

(57) **Abstract**

Provided is a method for measuring an anti-drug antibody that can be performed more simply and inexpensively than conventional methods. Provided is a double antigen bridging immunoassay using a capture nucleic acid and a tracer nucleic acid. By using the capture nucleic acid and the tracer nucleic acid in the double antigen bridging immunoassay, an anti-drug antibody can be measured simply and inexpensively. Furthermore, by using the tracer nucleic acid, it becomes possible to adopt a high sensitivity detection method utilizing a nucleic acid.

SEQUENCE LISTING

<110> SEKISUI MEDICAL CO., LTD.

<120> ANTIDRUG ANTIBODY MEASUREMENT METHOD

<130> 21P00236

<160> 4

<170> PatentIn version 3.5

<210> 1
<211> 22
<212> DNA
<213> Artificial Sequence

<220>
<223> capture nucleic acid

<400> 1
tacgttatca gactgatgtt ga 22

<210> 2
<211> 61
<212> DNA
<213> Artificial Sequence

<220>
<223> tracer nucleic acid

<400> 2

tacgttatca gactgatgtt gagatataag gagtggatac cgatgaagga tataaggagt 60

g 61

<210> 3
<211> 39
<212> DNA
<213> Artificial Sequence

<220>
<223> probe

<400> 3
gtcctgattg ttgcttcatc ggtatccact ccttatatc 39

<210> 4
<211> 39
<212> DNA
<213> Artificial Sequence

<220>
<223> probe

<400> 4
caacaatcag gacgataccg atgaaggata taaggagtg 39

## Description

### TECHNICAL FIELD

The present invention relates to a method for measuring an anti-drug antibody and a kit for use in such a measurement method.

### BACKGROUND ART

As a method for measuring an anti-drug antibody, a double antigen bridging immunoassay using a capture drug antibody and a tracer drug antibody is known (Patent Document 1). The method of Patent Document 1 is characterized in that the capture drug antibody is a mixture of said drug antibody containing at least two of said drug antibodies that differ in the antibody site at which they are bound to a solid phase and have the same amino acid sequence, and the tracer drug antibody is a mixture of said drug antibody containing at least two of said drug antibodies that differ in the antibody site at which they are bound to a detectable label and have the same amino acid sequence.

However, in the method of Patent Document 1, as described above, it is necessary to prepare two or more drug antibodies that differ in the antibody site at which they are bound to the solid phase or the detectable label and have the same amino acid sequence respectively for the capture drug antibody and the tracer drug antibody, i.e., the total of four or more drug antibodies should be prepared. Therefore, a high cost and a long time are required for the preparation of the both antibodies. Also, the performance management of immunoassay using said drug antibodies and the quality control of reagents become complicated.

Therefore, there is a need for more simple and less expensive measurement method of an anti-drug antibody.

The present inventors found that an anti-drug antibody can be measured simply and inexpensively by using a capture nucleic acid and a tracer nucleic acid, and thus completed the present invention. The capture nucleic acid and the tracer nucleic acid can be chemically synthesized inexpensively in an extremely simple manner, and can also be easily modified in various ways. Furthermore, the performance management of assay and the quality control of reagents can be performed simply.

### CITATION LIST

### PATENT LITERATURE

Patent Document 1: JP4902674 B2
Patent Document 2: JP3267576 B2

### SUMMARY OF INVENTION

### TECHNICAL PROBLEM

The problem to be solved by the present invention is to provide a more simple and inexpensive method for measuring an anti-drug antibody than conventional methods.

### SOLUTION TO PROBLEM

For solving the problem of the present invention, a double antigen bridging immunoassay using a capture nucleic acid and a tracer nucleic acid is provided. That is, the present invention is constituted of the following configurations <Embodiment 1> to <Embodiment 20>.

### <Embodiment 1>

A method for detecting an anti-drug antibody in a sample, the method comprising:
(i) a step of bringing a sample, a capture nucleic acid, and a tracer nucleic acid into contact to form a complex of the capture nucleic acid, the anti-drug antibody, and the tracer nucleic acid (also referred to as "a capture nucleic acid-anti-drug antibody-tracer nucleic acid complex"),
   wherein the capture nucleic acid and the tracer nucleic acid each has an epitope to which the anti-drug antibody binds; and
(ii) a step of detecting the capture nucleic acid-anti-drug antibody-tracer nucleic acid complex.

### <Embodiment 2>

A method for detecting an anti-drug antibody in a sample, the method comprising:
(i) a step of bringing a sample, a capture nucleic acid, and a tracer nucleic acid into contact to form a capture nucleic acid-anti-drug antibody-tracer nucleic acid complex,
   wherein the capture nucleic acid and the tracer nucleic acid each has an epitope to which the anti-drug antibody binds; and
(ii) a step of detecting the capture nucleic acid-anti-drug antibody-tracer nucleic acid complex using a sensitization method.

### <Embodiment 3>

A method for detecting an anti-drug antibody in a sample, the method comprising:
(i) a step of bringing a sample, a capture nucleic acid, and a tracer nucleic acid into contact to form a capture nucleic acid-anti-drug antibody-tracer nucleic acid complex,
   wherein the capture nucleic acid and the tracer nucleic acid each has an epitope to which the anti-drug antibody binds;
(ii) a step of bringing the capture nucleic acid-anti-drug antibody-tracer nucleic acid complex into contact with a pair of self-assembling probes composed of first and second oligonucleotides to form a polymer complex of the capture nucleic acid-anti-drug antibody-tracer nucleic acid complex and an oligonucleotide polymer produced by self-assembly of the first and second oligonucleotides; and
(iii) a step of detecting the polymer complex.

### <Embodiment 4>

A method for detecting an anti-drug antibody in a sample, the method comprising:
(i) a step of bringing a sample, a capture nucleic acid having an epitope to which the anti-drug antibody binds, and a tracer nucleic acid having an epitope to which the anti-drug antibody binds into contact with each other,
   wherein, when the sample contains the anti-drug antibody, a capture nucleic acid-anti-drug antibody-tracer nucleic acid complex is formed;
(ii) a step of trapping the capture nucleic acid, and separating and removing the tracer nucleic acid which is in a state free from the complex without being involved in the formation of the complex; and
(iii) a step of detecting the anti-drug antibody in the sample by detecting the tracer nucleic acid remaining without being separated or removed after the separation and removal step.

### <Embodiment 5>

A method for detecting an anti-drug antibody in a sample, the method comprising:
(i) a step of bringing a sample, a capture nucleic acid having an epitope to which the anti-drug antibody binds, and a tracer nucleic acid having an epitope to which the anti-drug antibody binds into contact with each other,
   wherein, when the sample contains the anti-drug antibody, a capture nucleic acid-anti-drug antibody-tracer nucleic acid complex is formed;
(ii) a step of trapping the capture nucleic acid,
   wherein the trapping step is performed before, after, during, or simultaneously with the formation of the complex;
(iii) a step of trapping the capture nucleic acid, and separating and removing the tracer nucleic acid which is in a state free from the complex without being involved in the formation of the complex; and
(iv) a step of detecting the anti-drug antibody in the sample by detecting the tracer nucleic acid remaining without being separated or removed after the separation and removal step.

### <Embodiment 6>

The method according to embodiment 4 or 5, wherein the step of trapping the capture nucleic acid is to perform binding of the capture nucleic acid to a solid phase, and the separation and removal step is to perform washing of the solid phase to which the capture nucleic acid binds.

### <Embodiment 7>

The method according to any one of embodiments 1 to 6, wherein two or more Ig classes of anti-drug antibodies are detected by one measurement when an IgG, IgM, IgD, IgE, or IgA class antibody is present in the sample.

### <Embodiment 8>

The method according to any one of embodiments 1 to 7, wherein the epitopes to which the anti-drug antibody binds in the capture nucleic acid and the tracer nucleic acid are the same.

### <Embodiment 9>

The method according to any one of embodiments 1 to 8, wherein nucleic acid chain lengths of the capture nucleic acid and the tracer nucleic acid are identical or the tracer nucleic acid has a longer nucleic acid chain length than that of the capture nucleic acid by 1-mer to 60-mers.

### <Embodiment 10>

The method according to any one of embodiments 1 to 9, wherein the nucleic acid chain length of the tracer nucleic acid is longer than that of the capture nucleic acid by 10-mers to 50-mers.

### <Embodiment 11>

The method according to any one of embodiments 1 to 10, wherein the step of bringing the sample, the capture nucleic acid, and the tracer nucleic acid into contact comprises a first step of bringing the sample and the capture nucleic acid into contact and a second step of bringing a product of the first step and the tracer nucleic acid into contact with each other, and
wherein, when the sample contains the anti-drug antibody, a capture nucleic acid-anti-drug antibody complex is formed in the first step, and the capture nucleic acid-anti-drug antibody-tracer nucleic acid complex is formed in the second step.

### <Embodiment 12>

The method according to embodiment 11, wherein the first complex-forming step and the second complex-forming step are performed simultaneously.

### <Embodiment 13>

The method according to any one of embodiments 1 to 12, wherein one or both of the capture nucleic acid and the tracer nucleic acid are chemically modified nucleic acids.

### <Embodiment 14>

The method according to embodiment 13, wherein the chemically modified nucleic acid is a locked nucleic acid (LNA), a bridged nucleic acid (BNA), a phosphorothioate oligonucleotide, a morpholino oligonucleotide, a boranophosphate oligonucleotide, a 2'-O-methylated RNA (2'-OMe), a 2'-O-methoxyethylated RNA (2'-MOE), or a 2'-F-RNA.

### <Embodiment 15>

The method according to any one of embodiments 1 to 14, wherein the anti-drug antibody is an anti-nucleic acid therapeutics antibody.

### <Embodiment 16>

The method according to any one of embodiments 1 to 15, wherein the sample is a blood-derived component.

### <Embodiment 17>

A kit for detecting an anti-drug antibody in a sample, comprising:
(1) a capture nucleic acid;
(2) a tracer nucleic acid; and
(3) a pair of self-assembling probes composed of first and second oligonucleotides;
wherein the capture nucleic acid and the tracer nucleic acid each has an epitope to which the anti-drug antibody binds.

### <Embodiment 18>

The kit according to embodiment 17, wherein the epitopes of the capture nucleic acid and the tracer nucleic acid for the anti-drug antibody are the same.

### <Embodiment 19>

A method for immunologically measuring an anti-drug antibody in a sample using a double antigen bridging immunoassay including a capture nucleic acid and a tracer nucleic acid,
wherein the capture nucleic acid and the tracer nucleic acid each has an epitope to which the anti-drug antibody binds.

### <Embodiment 20>

The method or the kit according to any one of embodiments 1 to 19,
wherein the capture nucleic acid has a structure of a single-stranded DNA having a chain length of 15 to 30-mers,
the tracer nucleic acid has a constitution of
an oligonucleotide composed of a nucleic acid region X of a first oligonucleotide, a nucleic acid region Y of the first oligonucleotide, the nucleic acid region X of the first oligonucleotide, and a sequence same as the capture nucleic acid in order from the 5' end side, or
an oligonucleotide composed of a nucleic acid region Z of the first oligonucleotide, the nucleic acid region Y of the first oligonucleotide, the nucleic acid region Z of the first oligonucleotide, and the sequence same as the capture nucleic acid in order from the 5' end side,
wherein
the first oligonucleotide has a constitution of
an oligonucleotide composed of the nucleic acid region X, the nucleic acid region Y, and the nucleic acid region Z in order from the 5' end, and
the second oligonucleotide has a constitution of
an oligonucleotide composed of a nucleic acid region X' complementary to the nucleic acid region X, a nucleic acid region Y' complementary to the nucleic acid region Y, and a nucleic acid region Z' complementary to the nucleic acid region Z in order from the 5' end; and
a label contained in the tracer nucleic acid or a label contained in the first or second oligonucleotide is detected.

### ADVANTAGEOUS EFFECTS OF INVENTION

By using the capture nucleic acid and the tracer nucleic acid in the double antigen bridging immunoassay, it is possible to measure an anti-drug antibody simply and inexpensively. Moreover, by using the tracer nucleic acid, it becomes possible to adopt a high sensitivity detection method utilizing a nucleic acid such as a PCR method or PALSAR method (Patent Document 2).

### BRIEF DESCRIPTION OF DRAWINGS

[Fig. 1] Fig. 1 is a schematic process illustration showing an aspect of a basic process of the present invention.
[Fig. 2] Fig. 2 is a graph showing signal values of Example 1.
[Fig. 3] Fig. 3 is a graph showing signal values of Example 2.

### DESCRIPTION OF EMBODIMENTS

In this specification, unless otherwise specified, the terms "measurement method" and "detection method" are used in their broadest senses including the identical concept.

Therefore, the method of the present invention can be used as a measurement method, detection method, quantitative measurement method, or qualitative measurement method of an anti-drug antibody by measuring the strength of detected signals.

In this specification, the term "anti-drug antibody" means an antibody directed to a drug. Such an antibody may possibly be produced, for example, as an immunogenic reaction in patients receiving a drug during drug therapy. The "anti-drug antibody" to be measured is not particularly limited as long as it is contained in biological samples. Preferred are IgG, IgM, IgD, IgE, or IgA, and more preferred is IgG or IgM.

Examples of the above anti-drug antibody include an "anti-nucleic acid therapeutics antibody". Examples of the "nucleic acid therapeutics" in the "anti-nucleic acid therapeutics antibody" include known "siRNA", "miRNA", "antisence", "aptamer", "decoy", "ribozyme", "CpG oligo", and "others (PolyI: PolyC (double-stranded RNA) for the purpose of activating innate immunity, antigene, or the like)". Moreover, the "nucleic acid therapeutics" include "a gene transfer vector for use in genetic medicine", "a gene contained in a genetic vaccine", and further a medicine containing a nucleic acid chain as an active ingredient such as a polydeoxyribonucleotide compound including defibrotide sodium (CAS registration number: 83712-60-1). Additionally, the "nucleic acid therapeutics" means an oligo nucleic acid composed of two or more nucleotides, and a nucleic acid constituting the oligo nucleic acid may have a non-natural structure (so-called a nucleic acid analogue) other than a natural structure. However, the nucleic acid analogue such as 5-FU (5-fluorouracil) itself is not included in the "nucleic acid therapeutics" in the present invention. The "nucleic acid therapeutics" of the present invention may be a single-stranded nucleic acid or a double-stranded nucleic acid. Further, in the case of a double-stranded nucleic acid, it may be a hetero double-stranded nucleic acid. Note that, in the present specification, the term "nucleic acid" means a polymer of nucleotide, but may also refer to a nucleotide itself depending on the context.

When an anti-drug antibody is produced against the above "nucleic acid therapeutics", an epitope to which the anti-drug antibody binds in the nucleic acid therapeutics may be a base moiety, a sugar moiety, or a phosphate moiety of a specific nucleotide in the oligo nucleic acid. Further, the number of nucleic acids constituting the epitope to which an anti-drug antibody binds may be either a specific nucleotide alone, or a nucleic acid composed of two or more nucleotides (oligo nucleic acid). Furthermore, for preparing nucleic acid therapeutics from an oligo nucleic acid, strength control of complementary binding, biodegradability control, or modification for the purpose of control of DDS, such as modification of sugar or phosphate in the nucleotide described later, a cyclic structure or a hairpin structure in the oligo nucleic acid, inclusion of a molecule other than a nucleic acid in the sequence of the oligo nucleic acid, inclusion of a tertiary structure formed between a molecule other than said nucleic acid and the oligo nucleic acid, or addition of polyethylene glycol, can be conducted. In such a case, the epitope to which an anti-drug antibody binds may be said modification moiety.

A "sample" used in the detection method of the present invention is not particularly limited as long as it is a biologically derived component, and an anti-drug antibody can be present therein. The sample is preferably the whole blood, serum, plasma, lymph fluid, or saliva of a human, monkey, dog, swine, rat, guinea pig, or mouse, and particularly preferably a blood-derived component such as the whole blood, serum, or plasma of a human. These may be used after dilution with water or a buffer solution. Further, the sample of the present invention includes those obtained by diluting anti-drug antibodies at a known concentration with water, a buffer solution, or a biologically derived component containing no anti-drug antibody (for example, a blood-derived component), and adjusting the concentration.

The above-described sample may be subjected to a pretreatment as necessary. For example, the property of the anti-drug antibody in the sample is changed by mixing with an acid or a surfactant, or substances affecting formation of a capture nucleic acid-anti-drug antibody-tracer nucleic acid complex in the sample is separated and removed by filtration using a filter capable of sieving a specific molecular weight fraction.

The above buffer solution may be those generally used, and examples thereof include tris-hydrochloric acid, boric acid, phosphoric acid, acetic acid, citric acid, succinic acid, phthalic acid, glutaric acid, maleic acid, glycine, and salts thereof, Good's buffer solutions such as MES, Bis-Tris, ADA, PIPES, ACES, MOPSO, BES, MOPS, TES, and HEPES. Examples of water include RNase- and DNase-free water. Note that the water used in the preparation of a buffer solution, or the like, is also preferably RNase- or DNase-free water.

The "capture nucleic acid" and "tracer nucleic acid" used in the detection method of the present invention are not particularly limited as long as they can be chemically synthesized, and are preferably DNA (deoxyribonucleic acid), RNA (ribonucleic acid), PNA (peptide nucleic acid), or a chemically modified nucleic acid. Non-natural nucleotide or any modification group may be included.

Any site of a base moiety, a sugar moiety, or a phosphate moiety of the nucleic acid may be targeted for chemical modification. Examples of the above chemically modified nucleic acid include phosphorothioate modification (sulfuration), 2'-F modification, 2'-O-Methyl (2'-OMe) modification, 2'-O-Methoxyethyl (2'-MOE) modification, morpholino modification, LNA modification, BNA^{COC} modification, BNA^{NC} modification, ENA modification, and cEtBNA modification.

Among these, preferred is a locked nucleic acid (LNA), bridged nucleic acid (BNA), phosphorothioate oligonucleotide, morpholino oligonucleotide, boranophosphate oligonucleotide, 2'-O-methylated RNA (2'-OMe), 2'-O-methoxyethylated RNA (2'-MOE), or 2'-F-RNA.

The above nucleic acid may be either single-stranded or double-stranded.

As the "capture nucleic acid" and "tracer nucleic acid" used in the detection method of the present invention, the above-described "nucleic acid therapeutics" itself may be used. Further, as long as an epitope to which an anti-drug antibody binds is contained within the structure, they may have an optional sequence, respectively. The sequence of the "capture nucleic acid" and the sequence of the "tracer nucleic acid" may be identical or different with each other.

In the "capture nucleic acid" and "tracer nucleic acid" used in the detection method of the present invention, the epitope to which an anti-drug antibody binds can be identified, for example, by a method using an affinity sensor based on surface plasmon resonance (SPR) as a detection principle, or an antigen competition method. The "capture nucleic acid" and "tracer nucleic acid" of the present invention preferably have identical epitopes to which the anti-drug antibody binds.

The capture nucleic acid is one or two or more species, and may be a combination of two or more species having different sites for binding to a solid phase. Also, the tracer nucleic acid is one or two or more species, and may be a combination of two or more species.

The "capture nucleic acid" used in the detection method of the present invention may include chemical modification for binding to the solid phase described later. The "tracer nucleic acid" may include chemical modification for binding the label for detecting an anti-drug antibody described later.

Nucleic acid chain length of the "capture nucleic acid" and "tracer nucleic acid" in the present invention is not particularly limited. Nucleic acid therapeutics having the nucleic acid chain length of more than ten mers to several tens of mers have been reported, and the nucleic acid chain length of the "capture nucleic acid" and "tracer nucleic acid" in the present invention can be matched to these lengths. In addition, the nucleic acid chain length suitable for the detection method of the present invention can be appropriately designed by taking into consideration desired specificity, sensitivity, and the like in detection of an anti-drug antibody. Preferred examples include a case that the chain length of the capture nucleic acid and the tracer nucleic acid are identical, or a case that the chain length of the tracer nucleic acid is longer than that of the capture nucleic acid by 1-mer to 60-mers. More preferred examples include cases that the chain length of the tracer nucleic acid is longer than that of the capture nucleic acid by 5-mers to 55-mers, 10-mers to 50-mers, 15-mers to 45-mers, 20-mers to 40-mers, 25-mers to 35-mers, 25-mr to 40-mers, 25-mers to 45-mers, 25-mers to 50-mers, 30-mers to 40-mers, 30-mers to 45-mers, 30 to 50-mers, 35-mers to 45-mers, and 35-mers to 50-mers.

In the chain length of the above nucleic acids, a sequence derived from nucleic acid therapeutics containing an epitope to which an anti-drug antibody binds is included, wherein the epitope is the cause of the anti-drug antibody production. Moreover, the identical or analogous sequence as the epitope to which an anti-drug antibody binds, or a sequence without having an analogous structure as the epitope may be included. When the nucleic acid chain length of the tracer nucleic acid is longer than that of the capture nucleic acid, said longer portion of the nucleic acid chain length preferably has the identical or analogous sequence as the epitope to which an anti-drug antibody binds, or a sequence without having an analogous structure as the epitope.

Specific structures of the "capture nucleic acid" in the present invention include, but are not limited to, the following examples:
5'-(n)a-(epitope)-(n)b-(functional group)-3', wherein "n" represents any nucleotide, "a" and "b" each independently represents 0 or a natural number, provided that the requirements of the nucleic acid chain length described above are satisfied, "epitope" represents an epitope to which an anti-drug antibody binds, and "functional group" represents a functional group such as an amino group which modifies the capture nucleic acid;
5'-(n)a-(epitope)-(n)b-(adaptor)-3', wherein "n" represents any nucleotide, "a" and "b" each independently represents 0 or a natural number, provided that the requirements of the nucleic acid chain length described above are satisfied, "epitope" represents an epitope to which an anti-drug antibody binds, and "adaptor" represents an adaptor such as biotin which binds to the capture nucleic acid;
5'-(functional group)-(n)a-(epitope)-(n)b-3, wherein "n" represents any nucleotide, "a" and "b" each independently represents 0 or a natural number, provided that the requirements of the nucleic acid chain length described above are satisfied, "epitope" represents an epitope to which an anti-drug antibody binds, and "functional group" represents a functional group such as an amino group which modifies the capture nucleic acid; and
5'-(adaptor)-(n)a-(epitope)-(n)b-3', wherein "n" represents any nucleotide, "a" and "b" each independently represents 0 or a natural number, provided that the requirements of the nucleic acid chain length described above are satisfied, "epitope" represents an epitope to which an anti-drug antibody binds, and "adaptor" represents an adaptor such as biotin which binds to the capture nucleic acid.

Specific structures of the "tracer nucleic acid" in the present invention include, but are not limited to, the following examples:
5'-(n)c-(epitope)-(n)d-(functional group)-3', wherein "n" represents any nucleotide, "c" and "d" each independently represents 0 or a natural number, provided that the requirements of the nucleic acid chain length described above are satisfied, "epitope" represents an epitope to which an anti-drug antibody binds, and "functional group" represents a functional group such as an amino group for labeling the tracer nucleic acid;
5'-(n)c-(epitope)-(n)d-(label)-3', wherein "n" represents any nucleotide, "c" and "d" each independently represents 0 or a natural number, provided that the requirements of the nucleic acid chain length described above are satisfied, "epitope" represents an epitope to which an anti-drug antibody binds, and "label" represents a label such as digoxigenin which modifies the tracer nucleic acid;
5'-(n)c-(epitope)-(n)d-(tag sequence)-3', wherein "n" represents any nucleotide, "c" and "d" each independently represents 0 or a natural number, provided that the requirements of the nucleic acid chain length described above are satisfied, "epitope" represents an epitope to which an anti-drug antibody binds, and "tag sequence" represents a tag sequence which is partially the same as the sequence of one of a pair of self-assembling probes (which is partially complementary to the sequence of the other of the pair of self-assembling probes);
5'-(functional group)-(n)c-(epitope)-(n)d-3', wherein "n" represents any nucleotide, "c" and "d" each independently represents 0 or a natural number, provided that the requirements of the nucleic acid chain length described above are satisfied, "epitope" represents an epitope to which an anti-drug antibody binds, and "functional group" represents a functional group such as an amino group for labeling the tracer nucleic acid;
5'-(label)-(n)c-(epitope)-(n)d-3', wherein "n" represents any nucleotide, "c" and "d" each independently represents 0 or a natural number, provided that the requirements of the nucleic acid chain length described above are satisfied, "epitope" represents an epitope to which an anti-drug antibody binds, and "label" represents a label such as digoxigenin which modifies the tracer nucleic acid; and
5'-(tag sequence)-(n)c-(epitope)-(n)d-3', wherein "n" represents any nucleotide, "c" and "d" each independently represents 0 or a natural number, provided that the requirements of the nucleic acid chain length described above are satisfied, "epitope" represents an epitope to which an anti-drug antibody binds, and "tag sequence" represents a tag sequence which is partially the same as the sequence of one of a pair of self-assembling probes (which is partially complementary to the sequence of the other of the pair of self-assembling probes).

In one aspect, the above tag sequence has the structure of an oligonucleotide composed of a nucleic acid region X of a first oligonucleotide, a nucleic acid region Y of the first oligonucleotide, and the nucleic acid region X of the first oligonucleotide in order from the 5' end side; or an oligonucleotide composed of a nucleic acid region Z of the first oligonucleotide, the nucleic acid region Y of the first oligonucleotide, and the nucleic acid region Z of the first oligonucleotide in order from the 5' end side. The above first oligonucleotide has the structure of an oligonucleotide composed of the nucleic acid region X, the nucleic acid region Y, and the nucleic acid region Z in order from the 5' end side. The second oligonucleotide has the structure of an oligonucleotide composed of a nucleic acid region X' complementary to the above nucleic acid region X, a nucleic acid region Y' complementary to the above nucleic acid region Y, and a nucleic acid region Z' complementary to the above nucleic acid region Z.

In this specification, the terms "bring into contact" or "contact step" mean to place a certain substance and other substance adjacently each other to form a chemical bond such as a covalent bond, ionic bond, metal bond, or non-covalent bond between the substances. In one aspect of the present invention, the "contact" step of a sample with the capture nucleic acid and the tracer nucleic acid in the detection method of the present invention is performed by mixing a liquid sample, a solution containing the capture nucleic acid, and a solution containing the racer nucleic acid in any combination.

The "epitope to which an anti-drug antibody binds" contained in the capture nucleic acid and the tracer nucleic acid used in the detection method of the present invention is as described above. The epitope is not particularly limited as long as an anti-drug antibody can bind to, and is preferably a nucleic acid, polypeptide, carbohydrate chain, protein, polymer compound, middle molecular compound, low molecular compound, or a part thereof.

Examples of the "epitope to which an anti-drug antibody binds" include, but are not limited to, the following; 5-methylated cytosine, phosphorothioate nucleic acid, boranophosphate nucleic acid, morpholino nucleic acid, LNA, BNA, 2'-O-methylated RNA (2'-OMe), 2'-O-methoxyethylated RNA (2'-MOE), 2'-F-RNA, ENA (registered trademark) (2'-O,4'-C-ethylene-bridged nucleic acids), N-acetyl galactosamine (GalNAc) nucleic acid, and polyethylene glycol.

Examples of the "sensitization method" used in the detection method of the present invention include a PCR method, tyramide signal amplification method, enzyme sensitization method, branched DNA method, dendrimer method, and PALSAR method. Preferred is PALSAR method.

In this specification, the term "self-assembly / self-aggregation" means a state that a plurality of the first oligonucleotides hybridize to the second oligonucleotide to form a complex, and a state that a plurality of the second oligonucleotides hybridize to the first oligonucleotide to form a complex.

The "pair of self-assembling probes composed of the first and second oligonucleotides" used in the detection method of the present invention refers to oligonucleotides capable of forming an oligonucleotide polymer by a self-assembly reaction, in which the first oligonucleotide and the second oligonucleotide have complementary nucleotide sequence regions which are hybridizable to each other. Preferably, at least one of the first and second oligonucleotides is labeled with a labeling substance. Herein, in one aspect, "hybridizable" means the state of being completely complementary in said complementary nucleotide sequence regions. Further, in another aspect, "hybridizable" means that the nucleotide sequences are complementary except for one or two mismatches in said complementary nucleotide sequence regions.

It is also possible to label the pair of self-assembling probes with a labeling substance for detection in advance. Preferred examples of such a labeling substance include a radioactive isotope, biotin, digoxigenin, fluorescent substance, luminescent substance, pigment, or metal complex.

The labeling substance is preferably a ruthenium complex, biotin, or digoxigenin. The oligonucleotide labeling is preferably performed by labeling the 5' end or the 3' end.

The pair of "self-assembling" probes is more specifically described as oligonucleotides containing at least the nucleic acid region X, nucleic acid region Y, and nucleic acid region Z in order from the 5' end side, in the first oligonucleotide, and an oligonucleotide containing at least the nucleic acid region X' complementary to the above nucleic acid region X, the nucleic acid region Y' complementary to the above nucleic acid region Y, and the nucleic acid region Z' complementary to the above nucleic acid region Z, in the second oligonucleotide.

Examples of the "solid phase" when the capture nucleic acid is bound to a solid phase include insoluble microparticles, microbeads, fluorescent microparticles, magnetic particles, a microplate, a microarray, a microscope slide, and a substrate such as an electrically conductive substrate. Preferred are magnetic particles or an electrically conductive substrate.

Examples of the binding of the capture nucleic acid to the solid phase include a chemical bonding method, a biological interaction method, and a physical adsorption method. In the chemical bonding method, for example, when using a solid phase coated with a functional group such as a carboxyl group, the capture nucleic acid may be modified with a functional group such as an amino group in advance, and then a coupling reaction may be performed between the functional groups. In the biological interaction method, for example, the avidity between streptavidin coated to a solid phase and biotin bound to the capture nucleic acid in advance can be used. Furthermore, in the physical adsorption method, for example, when using a solid phase having a negative electrical charge, by labeling the capture nucleic acid with a substance having a positive electrical charge such as an amino group, the capture nucleic acid can be electrostatically adsorbed to the solid phase.

Examples of the functional group for modifying the capture nucleic acid include, but are not limited to, the following: an amino group, a carboxyl group, a thiol group, and a maleimide group.

The method of "washing" the solid phase to which the capture nucleic acid binds is not particularly limited. For example, an optional amount of lavage fluid is added to the solid phase, and left to stand still or gently shaken to separate and remove the solution in the solid phase. Examples of the separation and removal methods of the solution preferably include decantation, centrifugal separation, and aspiration.

The step of separating and removing the solution by "decantation" is generally performed by tilting the solid phase to remove the solution.

The step of separating and removing the solution by "centrifugal separation" is generally performed by centrifugation at 500 to 3000 × g for 0.2 to 5 minutes at 20 to 30°C, centrifugation at 800 to 1500 × g for 0.5 to 2 minutes at 23 to 28°C, and preferably centrifugation at 1000 × g for 1 minute at 25°C to generate a supernatant, and then removing the supernatant.

The step of separating and removing the solution by "aspiration" is generally performed by using a micropipette or an aspirator. More specifically, the micropipette or aspirator may be used in accordance with the manufacturer's instruction manual.

After the capture nucleic acid-anti-drug antibody-tracer nucleic acid complex is formed, for "separating and removing" the tracer nucleic acid which is in the free state without being involved in the formation of said complex from the capture nucleic acid-anti-drug antibody-tracer nucleic acid complex, decantation, centrifugal separation, or aspiration is preferably used. Specific separation and removal methods are the same as the methods of separating and removing the solution. Examples of methods for detecting the tracer nucleic acid include a turbidity method, absorbance method, fluorophotometry, electrochemiluminescence method, and flow cytometry. Preferred is an electrochemiluminescence method.

The electrochemiluminescence method is performed by, for example, applying electric energy to an electrically conductive substrate to which the capture nucleic acid-anti-drug antibody-tracer nucleic acid complex binds, and detecting light emitted by reduction of a ruthenium complex which has been labeled to the tracer nucleic acid in advance.

In the present invention, the kit for detecting an anti-drug antibody in a sample includes at least the following components:
(1) a capture nucleic acid;
(2) a tracer nucleic acid; and
(3) a pair of self-assembling probes composed of the first and second oligonucleotides.

Herein, the capture nucleic acid and the tracer nucleic acid each has an epitope to which the above anti-drug antibody binds.

Each component is as described above.

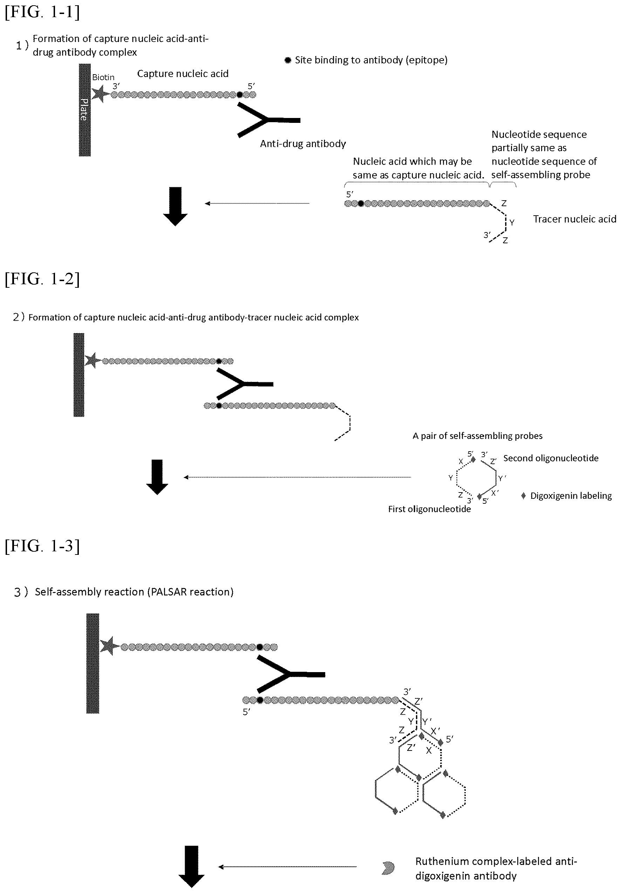

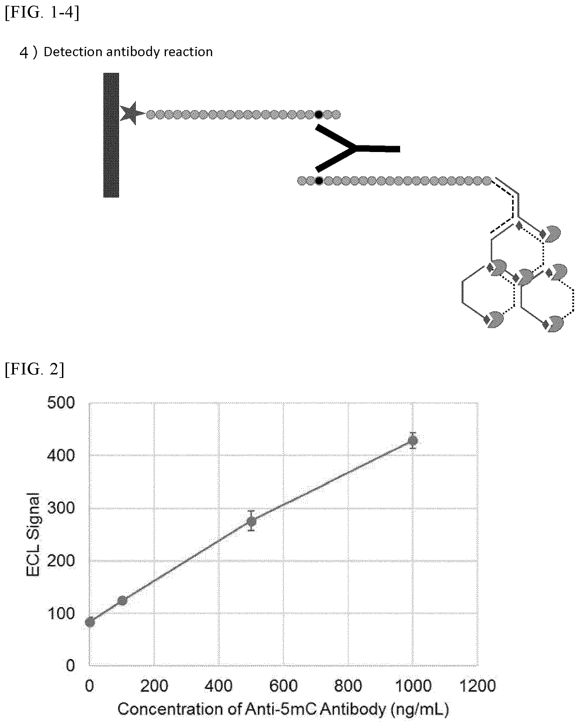

One aspect of the basic process of the present invention will be described with reference to Fig. 1.

In the beginning, a sample which may contain an anti-drug antibody, and a capture nucleic acid are provided. First, the capture nucleic acid modified with biotin (star mark in (Fig. 1-1)) is bound to a streptavidin-coated plate (solid phase). Then, the anti-drug antibody and the capture nucleic acid are brought into contact (Fig. 1-1). The anti-drug antibody binds to an epitope to which an anti-drug antibody binds in the capture nucleic acid, and thus a capture nucleic acid-anti-drug antibody complex is formed.

A tracer nucleic acid is brought into contact with the sample containing the above capture nucleic acid-anti-drug antibody complex (Fig. 1-2). In one embodiment, the tracer nucleic acid includes, at the 5' end or the 3' end, a tag sequence which is partially the same as the sequence of one of the pair of self-assembling probes (partially complementary to the sequence of the other of the pair of self-assembling probes). The anti-drug antibody binds to an epitope to which an anti-drug antibody binds in the tracer nucleic acid, and thus a capture nucleic acid-anti-drug antibody-tracer nucleic acid complex is formed.

It is possible to form the complexes in a different order than the one shown as an example in Fig. 1 which is (1) a complex of the capture nucleic acid and the anti-drug antibody is firstly formed, and then a complex with the tracer nucleic acid is formed. Specifically, it is also possible to: (2) firstly bring the anti-drug antibody into contact with the tracer nucleic acid to form an anti-drug antibody-tracer nucleic acid complex, and then bring a sample containing said complex into contact with the capture nucleic acid to form a capture nucleic acid-anti-drug antibody-tracer nucleic acid complex, or (3) bring the capture nucleic acid, the anti-drug antibody, and the tracer nucleic acid into contact with each other almost simultaneously to form a complex of these three components. In the above processes (1) to (3), the step of binding the capture nucleic acid to the solid phase can be performed at an appropriate timing, i.e., before, after, during, or simultaneously with the formation of the complex of two components or the complex of three components. The step of forming the complex of three components by bringing the three components into contact with each other almost simultaneously is preferably performed before binding the capture nucleic acid to the solid phase such as a plate.

In one embodiment, a sample containing the capture nucleic acid-anti-drug antibody-tracer nucleic acid complex is brought into contact with a pair of self-assembling probes composed of the first and second oligonucleotides to form a complex of the capture nucleic acid-anti-drug antibody-tracer nucleic acid complex and an oligonucleotide polymer produced by self-assembly of the first and second oligonucleotides (so called PALSAR reaction, Fig. 1-3).

Herein, in the pair of self-assembling probes composed of the first and second oligonucleotides, the first oligonucleotide contains the nucleic acid region X, the nucleic acid region Y, and the nucleic acid region Z in order from the 5' end side, and the second oligonucleotide contains the nucleic acid region X', the nucleic acid region Y', and the nucleic acid region Z' in order from the 5' end side. X and X', Y and Y', and Z and Z' are complementary to each other (hereinafter, said nucleic acid regions are sometimes simply referred to as X, Y, Z, X', Y', and Z'). In addition, the 5'-end of the first and second oligonucleotides are labeled with digoxigenin (diamond shapes in (Fig. 1-3)). Y' and Z' of the second oligonucleotide hybridize to Y and Z of the tracer nucleic acid in the capture nucleic acid-anti-drug antibody-tracer nucleic acid complex, and X', Y', and Z' of the second oligonucleotide hybridize to X, Y, and Z of the first oligonucleotide. Then, X and X', Y and Y', and Z and Z' are hybridized repeatedly one after another, an oligonucleotide polymer of the self-assembling probes is formed.

In one embodiment, a sample containing a complex of the capture nucleic acid-anti-drug antibody-tracer nucleic acid complex and the self-assembled first and second oligonucleotides is brought into contact with a ruthenium complex-labeled anti-digoxigenin antibody (Fig. 1-4). By detecting light emitted from the ruthenium complex, it is possible to measure a concentration of the anti-drug antibody, and the like.

It is also possible to detect the anti-drug antibody by detecting the capture nucleic acid-anti-drug antibody-tracer nucleic acid complex before conducting PALSAR reaction. Examples of a method for detecting said complex include, but are not limited to, the following.
(1) A ruthenium complex is bound to the tracer nucleic acid in advance, and the complex is detected directly.
(2) Digoxigenin is bound to a tracer nucleic acid in advance, and the complex is detected with a Ru complex-labeled anti-digoxigenin antibody.
(3) The following (A) is bound to the tracer nucleic acid in advance, and the complex is detected.

### (A): a radioactive isotope such as ¹²⁵I and ³²P, or a fluorescent dye such as Cyanin 5 and fluorescein

Specific examples of PALSAR method are shown in Figs. 4 to 14 of WO 2013/172305 or the like. They may be modified as appropriate for the application in the self-assembly reaction of the present invention based on common general knowledge of a person skilled in the art, by using a probe for dimer formation or the like.

The method of the present invention has extremely high specificity because the capture nucleic acid-anti-drug antibody-tracer nucleic acid complex is formed via epitopes to which the anti-drug antibody binds in the capture nucleic acid and the tracer nucleic acid. Further, the Ig class of the anti-drug antibody involved in formation of the complex is not limited. Thereby, when anti-drug antibodies of IgG, IgM, IgD, IgE, or IgA class are present, two or more Ig classes can be detected by one measurement. Herein, "one measurement" means that the contact of the sample, the capture nucleic acid, and the tracer nucleic acid is once. With this feature, the detection method of the present invention can sensitively detect the presence of an anti-drug antibody regardless of dosage interval or dosage period of a drug (nucleic acid therapeutics), or sample collection time, without an influence of Ig class switching.

According to the above, the present invention may be used for data acquisition to determine dosage regimen of nucleic acid therapeutics in an individual receiving the nucleic acid therapeutics, confirmation of the possibility of production of an anti-nucleic acid therapeutics antibody in development of the anti-nucleic acid therapeutics, and design of nucleic acid therapeutics itself by identifying an epitope to which said anti-drug antibody binds.

Hereinafter, specific aspects will be described in Examples for more easily understanding the present invention. However, the present invention is not limited to these Examples.

### EXAMPLES

### [Example 1]

### 1. Material and method

### (1) Target antibody

As an antibody to be measured, an anti-5-methylcytosine antibody (anti-5-mC antibody) (Abcam plc., product number ab10805) was used. The above target antibody was used after preparation to 0.1, 0.5, and 1 µg/mL with an antibody diluent. A blank sample containing no target antibody was also prepared.

### (1-1) Composition of antibody diluent

1×PBS-TP [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300], and 1% BSA (bovine serum albumin)

### (2) Capture nucleic acid

As a capture nucleic acid, the below-mentioned M-DNA1-3B produced by biotin modification at the 3' end of M-DNA1, which was used by Hasegawa et al. (Anal Sci. 2016;32(6):603-6), was used. The sequence of M-DNA1-3B is 5'-TACGTTATCAGACTGATGTTGA-3' (SEQ ID NO:1). Synthesis of the nucleic acid was requested to Nihon Gene Research Laboratories Inc. (HPLC purification grade). The third base from the 5' end (cytosine) in the above nucleic acid is 5-methylcytosine as in Hasegawa et al. M-DNA1-3B was used after preparation to 1 pmol/µL with TE (10 mM Tris-HCl (pH 8.0), 1 mM EDTA (pH 8.0)).

### (3) Tracer nucleic acid

As a tracer nucleic acid, the tracer nucleic acid (AP-ADA-M-DNA1-ZYZ-3N) which has the NH₂-modified 3' end and is composed of the nucleotide sequence same as the capture nucleic acid (22 nucleotides) and a nucleotide sequence which is partially complementary to a probe for signal amplification (HCP-2) (nucleotide sequence which is partially the same as a probe for signal amplification (HCP-1)) was used. Synthesis of the nucleic acid was requested to Nihon Gene Research Laboratories Inc. (HPLC purification grade). The third base from the 5' end (cytosine) in the above nucleic acid is 5-methylcytosine as in Hasegawa et al.

### <Sequence of AP-ADA-M-DNA1-ZYZ-3N>

5′-
TACGTTATCAGACTGATGTTGAGATATAAGGAGTGGATACCGATGAAGG
ATATAAGGAGTG -(NH2)-3′ (nucleotides portion corresponds to SEQ ID NO:2)

### (4) Formation of capture nucleic acid-target antibody-tracer nucleic acid complex (bridging reaction)

To 50 µL of target antibody, 50 µL of bridging reaction solution was added to obtain a 100 µL mixture in total. The mixture was allowed to react at 37°C for 3 hours while stirring at 600 rpm.

### (4-1) Composition of bridging reaction solution

1×PBS-TP [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300] 49.8 µL, 10 pmol/µL AP-ADA-M-DNA1-ZYZ-3N 0.1 µL, and 1 pmol/µL M-DNA1-3B 0.1 µL

### (5) Blocking of measurement plate

One hundred and fifty (150) µL of blocking solution was added to a streptavidin-immobilized measurement plate (Meso Scale Diagnostics, LLC., product number L45SA-1), and allowed to react at 25°C for 1 hour while stirring at 600 rpm. Then, the plate was washed twice with 200 µL of 1×PBS-TP.

### (5-1) Composition of blocking solution

1×PBS-TP [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300], and 3% BSA

### (5-2) Composition of 1×PBS-TP

1×PBS [137 mM sodium chloride, 8. 1 mM disodium phosphate, 2.68 mM potassium chloride, 1.47 mM potassium dihydrogenphosphate], 0.02% Tween 20, and 1.5 ppm ProClin 300

### (6) Fixation of capture nucleic acid-target antibody-tracer nucleic acid complex to measurement plate

After the bridging reaction, 75 µL of the reaction solution was added to the measurement plate after blocking, and allowed to react at 25°C for 1 hour while stirring at 600 rpm. Then, the plate was washed twice with 200 µL of 1×PBS-TP.

### (6-1) Composition of 1×PBS-TP

1×PBS [137 mM sodium chloride, 8.1 mM disodium phosphate, 2.68 mM potassium chloride, 1.47 mM potassium dihydrogenphosphate], 0.02% Tween 20, and 1.5 ppm ProClin 300

### (7) Detection assisting reaction

Fifty (50) µL of detection assisting reaction solution was added to the measurement plate after fixation of the capture nucleic acid-target antibody-tracer nucleic acid complex, and allowed to react at 40°C for 1 hour while stirring at 600 rpm. Then, the plate was washed twice with 200 µL of 1×PBS-TP.

### (7-1) Composition of detection assisting solution

Hybridization solution [189.3 mM Tris-HCl (pH 7.5), 2.4×PBS [328.8 mM sodium chloride, 19.44 mM disodium phosphate, 6.432 mM potassium chloride, 3.528 mM potassium dihydrogenphosphate], 3.6 mM EDTA (pH 8.0), 0.12% Tween 20] 21 µL, 10×supplement-T [500 mM Tris-HCl (pH 8.0), 40 mM EDTA (pH 8.0), 8% Tween 20, 1.5 ppm ProClin 300] 8µL, 20 pmol/µL HCP-2 0.42 µL, and Nuclease-Free Water 20.46 µL

### (7-2) Composition of 1×PBS-TP

1×PBS [137 mM sodium chloride, 8.1 mM disodium phosphate, 2.68 mM potassium chloride, 1.47 mM potassium dihydrogenphosphate], 0.02% Tween 20, and 1.5 ppm ProClin 300

### (7-3) Nucleotide sequence of HCP-2

The nucleic acid probe (HCP-2) used in Example 1 includes a sequence partially complementary to the nucleotide sequence of AP-ADA-M-DNA1-ZYZ-3N in the pair of self-assembling probes, and the 5' end thereof is labeled with digoxigenin.

### <Nucleotide sequence of HCP-2>

5'-(DIG)- GTCCTGATTGTTGCTTCATCGGTATCCACTCCTTATATC - 3' (nucleotides portion corresponds to SEQ ID NO:3)

### (8) Detection antibody reaction

Fifty (50) µL of detection antibody-reaction solution was added to the measurement plate after the detection assisting reaction, and allowed to react at 30°C for 30 minutes while stirring at 600 rpm. Then, the plate was washed twice with 200 µL of 1×PBS-TP.

### (8-1) Composition of detection antibody-reaction solution

1×PBS-TP (1% BSA) [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300, 1% BSA] 49.7 µL, and 100 µg/mL Ru-Anti-DIG, Fab fragment 0.3 µL

### (8-2) Preparation of Ru-Anti-DIG, Fab fragment

Ru-Anti-DIG, Fab fragment was prepared by binding MSD GOLD SULFO-TAG NHS-Ester (Meso Scale Diagnostics, LLC., product number R91AO-1) to Anti-Digoxigenin, Fab fragments (Merck KGaA, product number 11214667001) via a primary amine. The binding method was in accordance with the protocol of Meso Scale Diagnostics, LLC, attached to the product.

### (8-3) Composition of 1×PBS-TP

1×PBS [137 mM sodium chloride, 8.1 mM disodium phosphate, 2.68 mM potassium chloride, 1.47 mM potassium dihydrogenphosphate], 0.02% Tween 20, and 1.5 ppm ProClin 300

### (9) Detection

To the measurement plate after the detection antibody reaction, 150 µL of 0.5×Read Buffer [two-fold dilution of [MSD GOLD Read Buffer A (Meso Scale Diagnostics, LLC., product number R92TG-1)] with sterile purified water] was added, and light emission amount from the Ru-Anti-DIG, Fab fragment was measured using Meso QuickPlex SQ 120 MM system (manufactured by Meso Scale Diagnostics, LLC.) to detect signals of the target antibody.

### (10) Result

The measurement results are shown in Fig. 2. The signal increased with rise of concentration of the anti-5-methylcytosine antibody.

### [Example 2]

### 1. Material and method

### (1) Target antibody

As an antibody to be measured, the anti-5-methylcytosine antibody used in Example 1 (anti-5-mC antibody) (Abcam plc., product number ab10805) was used. The above target antibody was used after preparation to 0.1, 0.5, and 1 µg/mL with an antibody diluent. A blank sample containing no target antibody was also prepared.

### (1-1) Composition of antibody diluent

1×PBS-TP [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300], and 1% BSA

### (2) Capture nucleic acid

As a capture nucleic acid, M-DNA1-3B used in Example 1 was used. M-DNA1-3B was used after preparation at 1 pmol/µL with TE (10 mM Tris-HCl (pH 8.0), 1 mM EDTA (pH 8.0)).

### (3) Tracer nucleic acid

As a tracer nucleic acid, AP-ADA-M-DNA1-ZYZ-3N used in Example 1 was used.

### (4) Formation of capture nucleic acid-target antibody-tracer nucleic acid complex (bridging reaction)

To 50 µL of target antibody, 50 µL of bridging reaction solution was added to obtain a 100 µL mixture in total. The mixture was allowed to react at 37°C for 3 hours while stirring at 600 rpm.

### (4-1) Composition of bridging reaction solution

1×PBS-TP [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300] 49.8 µL, 10 pmol/µL AP-ADA-M-DNA1-ZYZ-3N 0.1 µL, and 1 pmol/µL M-DNA1-3B 0.1 µL

### (5) Blocking of measurement plate

One hundred and fifty (150) µL of blocking solution was added to a streptavidin-immobilized measurement plate (Meso Scale Diagnostics, LLC., product number L45SA-1), and allowed to react at 25°C for 1 hour while stirring at 600 rpm. Then, the plate was washed twice with 200 µL of 1×PBS-TP.

### (5-1) Composition of blocking solution

1×PBS-TP [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300], and 3% BSA

### (5-2) Composition of 1×PBS-TP

1×PBS [137 mM sodium chloride, 8.1 mM disodium phosphate, 2.68 mM potassium chloride, 1.47 mM potassium dihydrogenphosphate], 0.02% Tween 20, and 1.5 ppm ProClin 300

### (6) Fixation of capture nucleic acid-target antibody-tracer nucleic acid complex to measurement plate

Seventy five (75) µL of reaction solution after the bridging reaction was added to the measurement plate after the blocking, and allowed to react at 25°C for 1 hour while stirring at 600 rpm. Then, the plate was washed twice with 200 µL of 1×PBS-TP.

### (6-1) Composition of 1×PBS-TP

1×PBS [137 mM sodium chloride, 8.1 mM disodium phosphate, 2.68 mM potassium chloride, 1.47 mM potassium dihydrogenphosphate], 0.02% Tween 20, and 1.5 ppm ProClin 300

### (7) Self-assembly reaction (PALSAR reaction)

Fifty (50) µL of PALSAR reaction solution was added to the measurement plate after the fixation of the capture nucleic acid-target antibody-tracer nucleic acid complex, and allowed to react at 40°C for 1 hour while stirring at 600 rpm. Then, the plate was washed twice with 200 µL of 1×PBS-TP.

The sequences of a pair of self-assembling probes (also referred to as a probe for signal amplification) used in this Example are the following HCP-1 and HCP-2, in which the 5' ends thereof are labeled with digoxigenin.

### <Sequence of HCP-1>

5'-(DIG)-CAACAATCAGGACGATACCGATGAAGGATATAAGGAGTG -3' (nucleotides portion corresponds to SEQ ID NO:4)

### <Nucleotide sequence of HCP-2>

5'-(DIG)- GTCCTGATTGTTGCTTCATCGGTATCCACTCCTTATATC - 3' (nucleotides portion corresponds to SEQ ID NO:3)

### (7-1) Composition of PALSAR reaction solution

Hybridization solution [189.3 mM Tris-HCl (pH 7.5), 2.4×PBS [328.8 mM sodium chloride, 19.44 mM disodium phosphate, 6.432 mM potassium chloride, 3.528 mM potassium dihydrogenphosphate], 3.6 mM EDTA (pH 8.0), 0.12% Tween 20] 21 µL, 10×supplement-T [500 mM Tris-HCl (pH 8.0), 40 mM EDTA (pH 8.0), 8% Tween 20, 1.5 ppm ProClin 300] 8µL, 20 pmol/µL HCP-1 0.35 µL, 20 pmol/µL HCP-2 0.42 µL, and Nuclease-Free Water 20.11 µL

### (7-2) Composition of 1×PBS-TP

1×PBS [137 mM sodium chloride, 8.1 mM disodium phosphate, 2.68 mM potassium chloride, 1.47 mM potassium dihydrogenphosphate], 0.02% Tween 20, and 1.5 ppm ProClin 300

### (8) Detection antibody reaction

Fifty (50) µL of detection antibody-reaction solution was added to the measurement plate after the self-assembly reaction, and allowed to react at 30°C for 30 minutes while stirring at 600 rpm. Then, the plate was washed twice with 200 µL of 1×PBS-TP.

### (8-1) Composition of detection antibody-reaction solution

1×PBS-TP (1% BSA) [1×PBS [137 mM sodium chloride, 8.1 mM disodium phosphate, 2.68 mM potassium chloride, 1.47 mM potassium dihydrogenphosphate], 0.02% Tween 20, 1.5 ppm ProClin 300, 1% BSA] 49.7 µL, and 100 µg/mL Ru-Anti-DIG, Fab fragment 0.3 µL

### (8-2) Preparation of Ru-Anti-DIG, Fab fragment

Ru-Anti-DIG, Fab fragment was prepared by binding MSD GOLD SULFO-TAG NHS-Ester (Meso Scale Diagnostics, LLC., product number R91AO-1) to Anti-Digoxigenin, Fab fragments (Merck KGaA, product number 11214667001) via a primary amine. The binding method was in accordance with the protocol of Meso Scale Diagnostics, LLC., attached to the product.

### (8-3) Composition of 1×PBS-TP

1×PBS [137 mM sodium chloride, 8.1 mM disodium phosphate, 2.68 mM potassium chloride, 1.47 mM potassium dihydrogenphosphate], 0.02% Tween 20, and 1.5 ppm ProClin 300

### (9) Detection

To the measurement plate after the detection antibody reaction, 150 µL of 0.5×Read Buffer [two-fold dilution of [MSD GOLD Read Buffer A (Meso Scale Diagnostics, LLC., product number R92TG-1)] with sterile purified water] was added, and light emission amount from the Ru-Anti-DIG, Fab fragment was measured using Meso QuickPlex SQ 120 MM system (manufactured by Meso Scale Diagnostics, LLC.) to detect signals of the target antibody.

### (10) Result

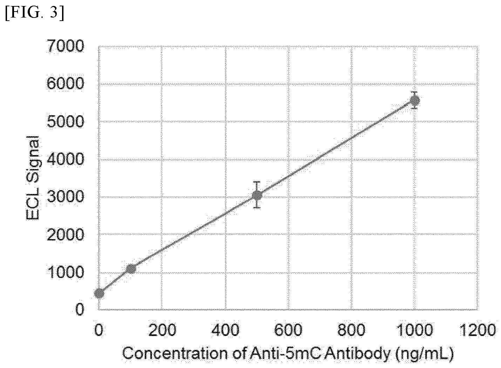

The measurement results are shown in Fig. 3. The signal increased with rise of concentration of the anti-5-methylcytosine antibody. From the results of Examples 1 to 2 described above, it was confirmed that the method of the present invention can detect an anti-drug antibody.

### INDUSTRIAL APPLICABILITY

The detection method of the present invention can be used simply and inexpensively for detecting an anti-drug antibody contained in a biologically derived sample, and for designing and producing a reagent or kit for performing said detection method.

## Claims

1. A method for detecting an anti-drug antibody in a sample, the method comprising:
(i) a step of bringing a sample, a capture nucleic acid, and a tracer nucleic acid into contact to form a complex of the capture nucleic acid, the anti-drug antibody, and the tracer nucleic acid (also referred to as "a capture nucleic acid-anti-drug antibody-tracer nucleic acid complex"),
wherein the capture nucleic acid and the tracer nucleic acid each has an epitope to which the anti-drug antibody binds; and
(ii) a step of detecting the capture nucleic acid-anti-drug antibody-tracer nucleic acid complex.

2. A method for detecting an anti-drug antibody in a sample, the method comprising:
(i) a step of bringing a sample, a capture nucleic acid, and a tracer nucleic acid into contact to form a capture nucleic acid-anti-drug antibody-tracer nucleic acid complex,
wherein the capture nucleic acid and the tracer nucleic acid each has an epitope to which the anti-drug antibody binds; and
(ii) a step of detecting the capture nucleic acid-anti-drug antibody-tracer nucleic acid complex using a sensitization method.

3. A method for detecting an anti-drug antibody in a sample, the method comprising:
(i) a step of bringing a sample, a capture nucleic acid, and a tracer nucleic acid into contact to form a capture nucleic acid-anti-drug antibody-tracer nucleic acid complex,
wherein the capture nucleic acid and the tracer nucleic acid each has an epitope to which the anti-drug antibody binds;
(ii) a step of bringing the capture nucleic acid-anti-drug antibody-tracer nucleic acid complex into contact with a pair of self-assembling probes composed of first and second oligonucleotides to form a polymer complex of the capture nucleic acid-anti-drug antibody-tracer nucleic acid complex and an oligonucleotide polymer produced by self-assembly of the first and second oligonucleotides; and
(iii) a step of detecting the polymer complex.

4. A method for detecting an anti-drug antibody in a sample, the method comprising:
(i) a step of bringing a sample, a capture nucleic acid having an epitope to which the anti-drug antibody binds, and a tracer nucleic acid having an epitope to which the anti-drug antibody binds into contact with each other,
wherein, when the sample contains the anti-drug antibody, a capture nucleic acid-anti-drug antibody-tracer nucleic acid complex is formed;
(ii) a step of trapping the capture nucleic acid, and separating and removing the tracer nucleic acid which is in a state free from the complex without being involved in the formation of the complex; and
(iii) a step of detecting the anti-drug antibody in the sample by detecting the tracer nucleic acid remaining without being separated or removed after the separation and removal step.

5. A method for detecting an anti-drug antibody in a sample, the method comprising:
(i) a step of bringing a sample, a capture nucleic acid having an epitope to which the anti-drug antibody binds, and a tracer nucleic acid having an epitope to which the anti-drug antibody binds into contact with each other,
wherein, when the sample contains the anti-drug antibody, a capture nucleic acid-anti-drug antibody-tracer nucleic acid complex is formed;
(ii) a step of trapping the capture nucleic acid,
wherein the trapping step is performed before, after, during, or simultaneously with the formation of the complex;
(iii) a step of trapping the capture nucleic acid, and separating and removing the tracer nucleic acid which is in a state free from the complex without being involved in the formation of the complex; and
(iv) a step of detecting the anti-drug antibody in the sample by detecting the tracer nucleic acid remaining without being separated or removed after the separation and removal step.

6. The method according to claim 4 or 5, wherein the step of trapping the capture nucleic acid is to perform binding of the capture nucleic acid to a solid phase, and the separation and removal step is to perform washing of the solid phase to which the capture nucleic acid binds.

7. The method according to any one of claims 1 to 6, wherein two or more Ig classes of anti-drug antibodies are detected by one measurement when an IgG, IgM, IgD, IgE, or IgA class antibody is present in the sample.

8. The method according to any one of claims 1 to 7, wherein the epitopes to which the anti-drug antibody binds in the capture nucleic acid and the tracer nucleic acid are the same.

9. The method according to any one of claims 1 to 8, wherein nucleic acid chain lengths of the capture nucleic acid and the tracer nucleic acid are identical or the tracer nucleic acid has a longer nucleic acid chain length than that of the capture nucleic acid by 1-mer to 60-mers.

10. The method according to any one of claims 1 to 9, wherein the nucleic acid chain length of the tracer nucleic acid is longer than that of the capture nucleic acid by 10-mers to 50-mers.

11. The method according to any one of claims 1 to 10, wherein the step of bringing the sample, the capture nucleic acid, and the tracer nucleic acid into contact comprises a first step of bringing the sample and the capture nucleic acid into contact with each other and a second step of bringing a product of the first step and the tracer nucleic acid into contact with each other, and
wherein, when the sample contains the anti-drug antibody, a capture nucleic acid-anti-drug antibody complex is formed in the first step, and the capture nucleic acid-anti-drug antibody-tracer nucleic acid complex is formed in the second step.

12. The method according to claim 11, wherein the first complex-forming step and the second complex-forming step are performed simultaneously.

13. The method according to any one of claims 1 to 12, wherein one or both of the capture nucleic acid and the tracer nucleic acid are chemically modified nucleic acids.

14. The method according to claim 13, wherein the chemically modified nucleic acid is a locked nucleic acid (LNA), a bridged nucleic acid (BNA), a phosphorothioate oligonucleotide, a morpholino oligonucleotide, a boranophosphate oligonucleotide, a 2'-O-methylated RNA (2'-OMe), a 2'-O-methoxyethylated RNA (2'-MOE), or a 2'-F-RNA.

15. The method according to any one of claims 1 to 14, wherein the anti-drug antibody is an anti-nucleic acid therapeutics antibody.

16. The method according to any one of claims 1 to 15, wherein the sample is a blood-derived component.

17. A kit for detecting an anti-drug antibody in a sample, comprising:
(1) a capture nucleic acid;
(2) a tracer nucleic acid; and
(3) a pair of self-assembling probes composed of first and second oligonucleotides;
wherein the capture nucleic acid and the tracer nucleic acid each has an epitope to which the anti-drug antibody binds.

18. The kit according to claim 17, wherein the epitopes of the capture nucleic acid and the tracer nucleic acid for the anti-drug antibody are the same.

19. A method for immunologically measuring an anti-drug antibody in a sample using a double antigen bridging immunoassay including a capture nucleic acid and a tracer nucleic acid,
wherein the capture nucleic acid and the tracer nucleic acid each has an epitope to which the anti-drug antibody binds.
